Europäisches Patentamt

(19) European Patent Office

Office européen des brevets

(11) Numéro de publication: **0 158 619**
A2

## (12) DEMANDE DE BREVET EUROPEEN

(21) Numéro de dépôt: **85870018.0**

(22) Date de dépôt: **05.02.85**

(51) Int. Cl.⁴: **A 61 B 17/42**
**A 61 B 19/04**

(30) Priorité: **09.02.84 BE 212358**

(43) Date de publication de la demande:
**16.10.85 Bulletin 85/42**

(84) Etats contractants désignés:
**AT CH DE FR GB IT LI LU NL SE**

(71) Demandeur: **Van den Boogaerde, André Robert**
**Nederpolder 7**
**B-9000 Gent(BE)**

(72) Inventeur: **Van den Boogaerde, André Robert**
**Nederpolder 7**
**B-9000 Gent(BE)**

(74) Mandataire: **Vigneron, Jean**
**Cabinet VIGNERON 30 avenue Eugène Godaux**
**B-1150 Bruxelles(BE)**

(54) Instrument chirurgical.

㊼ Instrument chirurgical 1 destiné, en particulier, à être introduit à travers le vagin pour rompre, en cours d'accouchement, la membrane ou sac amniotique, cet instrument 1 étant constitué par un gant 3 en matière souple et par un élément recourbé 4, en forme de griffe ou de crochet, réalisé en matière rigide et associé à l'extrémité 5 d'un des doigts 6 du gant 3.

La figure à considérer est la figure 2.

EP 0 158 619 A2

FIG. 2.

# INSTRUMENT CHIRURGICAL

La présente invention a pour objet un instrument chirurgical destiné, en particulier, à être introduit à travers le vagin pour rompre, en cours d'accouchement, la membrane ou sac amniotique.

En cours d'accouchement, la contraction du muscle utérin se répercute sur le sac, contenu dans la cavité utérine, renfermant le foetus et le liquide amniotique et exerce sur ce sac une force dirigée de haut en bas. Le sac ayant un volume irréductible du fait de la présence du liquide amniotique, il exerce une pression sur le segment inférieur de l'utérus où les tissus sont plus malléables et où il existe le canal cervical capable de céder, en se dilatant, sous l'effet de la force précitée. Quand les contractions progressent, le col se raccourcit jusqu'à sa totale disparition et la dilatation se poursuit graduellement jusqu'à atteindre un diamètre de 8 à 9 cm. C'est alors que se produit normalement la rupture des membranes ovulaires et une partie du liquide amniotique se déverse dans le vagin, les contractions du muscle utérin se répercutent alors, non plus sur le sac à volume irréductible, mais directement sur le foetus pour l'expulser de la cavité utérine vers le vagin et vers l'extérieur.

Dans certains cas, la rupture du sac amniotique ne s'effectue pas naturellement, et on est alors obligé, pour que l'accouchement puisse se poursuivre normalement, de provoquer manuellement la rupture du sac amniotique en agissant à travers le vagin.

- 2 -

0158619

Pour procéder à cette intervention, on utilise un instrument rigide, métallique ou en matière plastique, présentant une portion sensiblement rectiligne terminée par une extrémité recourbée que l'on introduit dans le vagin, en le guidant tant bien que mal à l'aide d'un doigt également introduit dans le vagin, jusqu'au contact du sac amniotique dans lequel on fait alors pénétrer l'extrémité recourbée de l'instrument pour provoquer sa rupture.

L'utilisation d'un tel instrument présente notamment l'inconvénient de nécessiter un très grand doigté, d'une part, pour manipuler l'instrument et, d'autre part, pour assurer son guidage précis si l'on veut éviter d'occasionner des lésions plus ou moins graves à la mère ou au foetus.

L'invention a pour but de remédier à cet inconvénient et de procurer un instrument extrêmement simple dont l'introduction dans le vagin jusqu'au sac amniotique peut être contrôlée de manière extrêmement précise à l'aide d'un seul doigt, tout comme son maniement, lorsqu'il est au contact du sac, pour rompre ce dernier, ce qui permet d'éviter les lésions précitées. De plus, le faible coût de l'instrument suivant l'invention permet de le jeter après usage, ce qui offre l'avantage, par rapport aux instruments métalliques connus, d'éviter les opérations de stérilisation.

A cet effet, suivant l'invention, l'instrument est constitué soit par un doigtier, soit par un gant en matière souple, du type utilisé en chirurgie et pour les manipulations, et par un élément recourbé, en forme de griffe ou de crochet et réalisé en matière rigide, qui est associé à l'extrémité du doigtier ou d'un des doigts du gant.

D'autres détails et particularités de l'invention ressortiront de la description des dessins annexés au présent mémoire et qui représentent, à titre d'exemples non limitatifs, deux formes de réalisation particulières de l'instrument chirurgical suivant l'invention.

- 3 -

0158619

La figure 1 est une vue en perspective de l'instrument chirurgical suivant l'invention.

La figure 2 est une vue en perspective d'une variante de l'instrument illustré à la figure 1.

Dans les différentes figures, les mêmes notations de référence désignent des éléments identiques ou analogues.

L'instrument chirurgical 1 suivant l'invention et représenté aux dessins est destiné, en particulier, à être introduit à travers le vagin, pour provoquer, lorsque celle -ci ne se produit pas naturellement, la rupture de la membrane ou sac amniotique afin de permettre l'écoulement du liquide amniotique par le vagin pour faciliter l'expulsion du foetus de la cavité utérine. Cet instrument 1 est constitué soit par un doigtier 2, comme montré à la figure 1 , soit par un gant 3, comme montré à la figure 1, en matière souple, du type que l'on utilise pour les manipulations et en chirurgie, et par un élément recourbé 4, en forme de griffe ou de crochet, cet élément 4 étant réalisé en matière rigide, telle qu'une matière plastique, qui est associé à l'extrémité 5 du doigtier 2 ou d'un des doigts 6 du gant 3. L'élément 4 est associé au doigtier ou au gant, pour que son extrémité effilée libre 7 soit tournée du côté de la paume de la main de sorte qu'en recourbant plus ou moins le doigt à l'extrémité duquel est situé l'élément , on peut faire pénétrer ce dernier dans le vagin avec une extrême précision jusqu'au sac amniotique et ce, en l'orientant de telle sorte que la pointe de l'élément ne puisse entrer directement en contact avec les parois du vagin. Lorsque l'élément 4 est au contact du sac amniotique, il suffit d'une simple flexion du doigt pouvant également être réalisée avec une extrême précision, pour faire pénétrer son extrémité effilée dans la paroi du sac et provoquer, lors du retrait dudit élément, la déchirure du sac amniotique.

Lorsqu'il est associé à un gant, l'élément recourbé 4 est avantageusement situé dans le prolongement du doigt du gant correspondant au médius.

L'élément 4 peut être constitué par une pièce rapportée en matière plastique rigide, qui est choisie pour autoriser l'assemblage,
par soudure ou collage , dudit élément sur le doigtier ou le doigt
du gant. L'élément 4 peut également être incorporé au gant ou au
doigtier lors de leur fabrication, de sorte qu'ils ne forment qu'une
seule pièce.

Il doit être entendu que l'invention n'est nullement limitée aux
formes de réalisation décrites et que bien des modifications peuvent
être apportées à ces dernières sans sortir du cadre du présent brevet.

REVENDICATIONS

_____

1) Instrument chirurgical (1) destiné, en particulier,à être introduit à travers le vagin pour rompre,
en cours d'accouchement, la membrane ou sac amniotique,
ledit instrument étant caractérisé en ce qu'il est constitué soit par un doigtier (2) , soit par un gant (3)
en matière souple, du type utilisé en chirurgie et pour
les manipulations, et par un élément recourbé (4) , en
forme de griffe ou de crochet et réalisé en matière rigide, qui est associé à l'extrémité (5) du doigtier (2) ou
d'un des doigts (6) du gant (3).

2) Instrument chirurgical suivant la revendication 1,
caractérisé en ce que l'élément recourbé (4) susdit est
associé au doigtier (2) ou au doigt (6) du gant (3) pour
que son extrémité libre (7) soit orientée du côté de la
paume de la main, de manière à ce que par une simple flexion
du doigt, recouvert du doigtier ou du doigt du gant portant
ledit élément et introduit dans le vagin,on puisse faire pénétrer l'extrémité libre (7) de cet élément (4) dans le sac
amniotique et rompre ce dernier lorsque l'on en dégage ledit
élément.

3) Instrument chirurgical suivant l'une ou l'autre des
revendications 1 et 2, caractérisé en ce que ledit élément
recourbé (4) est associé au doigt (6) du gant (3) correspondant au médius.

4) Instrument chirurgical suivant l'une quelconque des
revendications 1 à 3 , caractérisé en ce que l'élément recourbé (4) est une pièce rapportée,par soudure ou par collage, sur le doigtier (2) ou le doigt (6) du gant (3) précité.

5) Instrument chirurgical suivant l'une quelconque des
revendications 1 à 3, caractérisé en ce que le doigtier (2)
ou le gant (3) et l'élément recourbé (4) sont réalisés en
une seule pièce.

FIG.1.

FIG. 2.

FIG. 2.